(19) Europäisches Patentamt
European Patent Office
Office européen des brevets

(11) **EP 4 142 852 B1**

(12) **EUROPÄISCHE PATENTSCHRIFT**

(45) Veröffentlichungstag und Bekanntmachung des Hinweises auf die Patenterteilung:
**20.03.2024 Patentblatt 2024/12**

(21) Anmeldenummer: **21727548.6**

(22) Anmeldetag: **28.04.2021**

(51) Internationale Patentklassifikation (IPC):
**A61M 25/10** (2013.01)  **A61M 31/00** (2006.01)
**A61F 5/44** (2006.01)  **A61F 5/451** (2006.01)

(52) Gemeinsame Patentklassifikation (CPC):
(C-Sets verfügbar)
**A61L 29/041; A61L 29/06;** A61F 5/44;
A61M 25/1002; A61M 2202/068; A61M 2205/0238;
A61M 2210/1064; A61M 2210/1067  (Forts.)

(86) Internationale Anmeldenummer:
**PCT/IB2021/053539**

(87) Internationale Veröffentlichungsnummer:
**WO 2021/220197 (04.11.2021 Gazette 2021/44)**

(54) **KATHETERVORRICHTUNG FÜR DIE GERUCHSREDUZIERENDE ZU- UND/ODER ABLEITUNG VON SUBSTANZEN ZU/AUS DEM MENSCHLICHEN ODER TIERISCHEN KÖRPER**

CATHETER DEVICE FOR ODOUR-REDUCING DELIVERY AND/OR DISCHARGE OF SUBSTANCES TO/FROM THE HUMAN OR ANIMAL BODY

DISPOSITIF DE CATHÉTER POUR L'ADMINISTRATION ET/OU L'ÉVACUATION DE SUBSTANCES, EN RÉDUISANT LES ODEURS, VERS/À PARTIR DU CORPS HUMAIN OU ANIMAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2020 DE 102020002764**
**18.05.2020 PCT/IB2020/054684**

(43) Veröffentlichungstag der Anmeldung:
**08.03.2023 Patentblatt 2023/10**

(73) Patentinhaber: **Advanced Medical Balloons GmbH**
**68753 Waghäusel (DE)**

(72) Erfinder: **GÖBEL, Fred**
**67346 Speyer (DE)**

(74) Vertreter: **Küchler, Stefan**
**Stefan T. Küchler**
**Patentanwalt**
**Färberstraße 20**
**90402 Nürnberg (DE)**

(56) Entgegenhaltungen:
**WO-A1-2013/056013  US-A- 6 033 390**
**US-A1- 2013 060 212  US-A1- 2014 358 126**
**US-A1- 2015 282 978**

(52) Gemeinsame Patentklassifikation (CPC): (Forts.)

C-Sets
**A61L 29/041, C08L 27/06;**
**A61L 29/041, C08L 29/04;**
**A61L 29/06, C08L 75/04;**
**A61L 29/06, C08L 77/00;**
A61M 2202/068, A61M 2202/0014

# EP 4 142 852 B1

**Beschreibung**

Die Erfindung richtet sich auf eine katheterartige Vorrichtung für die medizinische Zuleitung und/oder Ableitung von besonders geruchsintensiven, besonders löslichen oder sonstig migrationsfähigen Substanzen, umfassend eine die in einem Organ oder in einem Körperbinnenraum platzierte Vorrichtung ankernd retinierende Ballonkomponente, eine sich an die Ballonkomponente anschließende, trans-luminale, zum Organ oder zum Binnenraum führende zu- und/oder ableitende Schlaucheinheit, sowie einen sich daran, nach extrakorporal anschließenden, schlauchartigen Anteil,

Katheter für die kontinuierliche Ableitung von Stuhl aus dem Rektum eines Patienten sind heute ein etablierter Bestandteil der intensivmedizinischen Versorgung. Stuhldrainagen konventioneller Bauart bestehen aus einem torusartigen Ballon, der den Katheter auf dem Boden des Rektums aufliegend verankert, sowie einem, den Ballontorus tragenden, durch den analen Schließmuskel hindurchreichenden stuhlableitenden Schlauchelement, welches in extrakorporaler Verlängerung mit einem dort angeordneten Sammelbehälter konnektiert.

Stuhlableitende Drainagevorrichtungen der beschriebenen einfachen Bauart sind konzeptionell nicht in der Lage dünnflüssige Stühle zuverlässig geschlossen, also ohne Austritt von Stuhl, durch den Schließmuskel hindurch abzuleiten. Eine Verschmutzung der perianalen Hautpartien sowie eine Kontamination der unmittelbaren Pflegeumgebung des Patienten kann nicht verhindert werden. Die Problematik der Dichtung des analen Schließmuskels bleibt im Design dieses Vorrichtungstyps vollständig unberücksichtigt. Das den Analkanal passierende, stuhlableitende Schlauchsegment geht bei normalem Tonus, des dem Schlauchsegment anliegenden Schließmuskels, in eine radiale Faltung über, wobei das Schlauchsegment innerhalb des Analkanals grobe, längsverlaufende Kanäle formiert, die flüssigen Darminhalt aus dem Rektum in den perianalen Bereich abfließen lassen. Um den trans-analen Abfluss von Stuhl zu begrenzen, wurde bei einigen Ausführungen dieses Bautyps ein besonders dünnwandiges und weiches, trans-anales Schlauchsegment integriert. Die resultierende radiale Faltung der Schlauchwandung stellt sich zwar weniger grob dar, die entsprechend ausgestatteten Drainagen begünstigen jedoch axiale Verwindungen des Schlauchsegmentes, die leicht zum vollständigen Verschluss des stuhlableitenden Lumens führen.

Konventionelle, stuhlableitende Drainagen bestehen in aller Regel sowohl im intrakorporalen, stuhlaufriehmenden Kopfteil, als auch, im stuhlableitenden, extrakorporalen Schlauchteil, vollständig aus Silikon.

Silikone sind insbesondere bei der Verarbeitung zu dünnwandigen Ballon- und Schlauchstrukturen nur sehr unzureichend in der Lage, die Freisetzung von geruchsintensiven Substanzen zu verhindern bzw. auf ein tolerables Maß zu reduzieren. Werden native, unbeschichtete oder sonstig unbehandelte silikonbasierte Komponenten beispielsweise flüssigem Stuhl exponiert, kann sich bereits nach wenigen Stunden an den zur Umgebung hin gerichteten Oberflächen äußerst intensiver Geruch entwickeln. Der vom System ausgehende Geruch kann sowohl für den Patienten als auch für den Anwender derart belastend sein, dass die kontinuierliche Stuhlableitung abgebrochen werden muss.

Hersteller statten silikonbasierte Stuhlableitungssysteme im Bereich des extrakorporalen Schlauchsegmentes daher häufig mit barriere-wirksamen, den Durchtritt von Geruchsstoffen hemmenden, Geruchsstoffe adsorbierenden oder Geruchsstoffe neutralisierenden Hilfsstoffen aus, um den Stuhlgeruch in einem akzeptablen Bereich zu halten.

US2014/358126 A1 offenbart eine Vorrichtung zur Abdichtung eines Dickdarms und zur Entfernung von Stuhl aus diesem durch Spülung, mit einem ausdehnbaren intrarektalen Ballonsegment, das aus einem umgedrehten Schlauch mit zwei Enden gebildet ist, zum Einführen in das Rektum zur Verankerung der Vorrichtung.

US 6,033,390 A offenbart eine Stomavorrichtung.

US2013/060212 A1 offenbart einen Stoma-Einsatz zum Leiten von Darminhalt aus einem Darmabschnitt in einer Bauchhöhle durch ein Stoma in einer Bauchwand.

US2015/282978 A1 offenbart eine geruchshemmende, mehrschichtige Barriereanordnung, die in zahlreichen medizinischen Anwendungen und insbesondere in Folien für Stomaprodukte verwendet werden kann.

WO2013/056013 A1 offenbart eine Polymerfolie, die mindestens eine geruchsabsorbierende Schicht mit einer geruchsabsorbierenden PVDC-Mischung enthält.

Seit einigen Jahren sind im Markt neuartige Stuhlableitungssysteme mit dichtungsoptimierten, trans-anal dichtenden Kopfeinheiten aus Polyurethan (PUR) bekannt, wie beispielsweise der Typ hyghtec® basic-plus, der Firma Creative Ballons GmbH, Waghäusel, Deutschland. Dieser Drainagetyp weist für die extrakorporale Stuhlableitung zum Sammelgefäß einen optional aus PUR oder PVC hergestellten, Ableitungsschlauch auf. Sowohl natives PUR als auch natives PVC, als auch Compounds oder Koextrusionen der beiden Materialien, erweisen sich bei längeren Verweilzeiten im Körper ebenfalls nur unzureichend geruchsdicht.

Während die trans-anal positionierte Kopfeinheit dieses neuartigen Bautyps nahezu vollständig innerhalb des Körpers des Patienten liegt, und lediglich ein kleines, sphärisch erweitertes Segment des trans-analen Ballonkorpus aus dem Anus herausragt, entwickelt die zum Sammelbeutel führende, stuhlableitende Schlaucheinheit eine deutlich größere, geruchswirksame Gesamtfläche von ca. 1400 cm$^2$, ausgehend von einem Schlauchdurchmesser von 2,5 cm und einer Schlauchlänge von 180 cm. Die stuhlableitende, extrakorporale Schlaucheinheit muss bei kontinuierlich stuhlableitenden Systemen als primäre Quelle für die Freisetzung von Stuhlgeruch betrachtet werden. Das sammelnde Gefäß, sowie der prä-anal sichtbare Ballonanteil spielen eine untergeordnete Rolle.

Aus diesen Nachteilen des bisherigen Standes der Technik resultiert das die Erfindung initiierende Problem, eine gattungsgemäße katheterartige Vorrichtung derart weiterzubilden, dass bei einem längeren, bspw, mehrtägigen Verbleib in einem Patienten kein Stuhlgeruch freigesetzt wird.

Die Erfindung ist im unabhängigen Anspruch 1 definiert. Die abhängigen Ansprüche betreffen bevorzugte Ausführungsformen der Erfindung.

Die Lösung geligt im Rahmen einer gattungsgemäßen katheterartigen Vorrichtung dadurch, dass wenigstens der extrakorporale, schlauchartige Anteil aus einer mehrlagigen Folie gebildet wird, wobei die mehrlagige Folie wenigstens eine Barriere-Lage aus einem geruchsdichtenden Barrierematerial umfasst sowie wenigstens eine Träger-Lage aus einem anderen, mechanisch stabilen Material, wobei die Wandstärke der Träger-Lage größer ist als die Wandstärke der Barriere-Lage.

Damit beschreibt die vorliegende Erfindung technische Lösungswege zur Eindämmung bzw. weitestgehend möglichen Vermeidung einer Geruchsfreisetzung bei der kontinuierlichen Ableitung von Stuhl oder auch von anderen geruchsintensiven Exkreten, Sekreten oder sonstigen Körperflüssigkeiten. Die Erfindung umfasst ferner auch die geruchsreduzierende bzw. geruchvermeidende Zuleitung von entsprechend geruchsintensiven Substanzen zum Körper.

Die Erfindung bietet vorzugsweise technische Lösungen für den extra-korporalen, zu- und/oder ableitende Schlauchanteil der Kathetervorrichtung, bezieht aber auch auf andere geruchsfreisetzende Oberflächen der Kathetervorrichtung und des Sammelbeutels.

Die beschriebene Schlauch- und Folientechnologie beruht auf der Verwendung mehrlagiger Materialkombinationen, wobei z.B. Polyurethan (PUR) bzw. thermoplastisches PUR (TPU), als besonders beständiges, mechanisch robustes Trägermaterial, mit EVOH (Ethylen-Vinyl-Copolymer) oder PVDC (Polyvinylidenchlorid), als jeweils bewährte, geruchsdichte Barriere-Materialien, zu mehrlagigen Folienstrukturen verbunden werden. Sowohl EVOH als auch PVDC sind aufgrund ihrer chemischen Eigenschaften nicht mit Silikon kombinierbar, insbesondere nicht als mehrlagig koextrudierte oder auch mehrlagig laminierte Folie herstellbar.

Die im Rahmen der vorliegenden Erfindung vorgestellten, mehrlagig aufgebauten Schlauchfolien umfassen eine oder mehrere Lagen eines mechanisch belastbaren, bevorzugt elastisch verformbaren und sich elastisch aufrichtenden Trägermaterials, wobei bevorzugt thermoplastische Polyurethane (TPU) verwendet werden. Weniger bevorzugt können auch PVC, Polyamid (PA), Pebax oder auch beispielsweise Polyolefin-basierte Polymere zur Anwendung kommen.

Das jeweilige Schlauchfolienmaterial kann beispielhaft aus einer sandwichartigen Kombination von PUR auf den innen und außen liegenden Seiten, und einer mittig angeordneten Lage aus EVOH oder PVCD aufgebaut sein. Während sich die Barriere-Eigenschaften von EVOH bei Exposition mit Wasser verschlechtern, widersteht PVDC einem wässrigem Milieu und kann daher optional auch in einem zwei-lagigen Materialverbund, mit beispielsweise PUR oder PVC, ohne Verlust seiner Barrierewirkung verwendet werden. Bei einem drei- oder mehrlagigen Folienaufbau können PVC und PUR, entweder als Innen- oder Außenlage, um die mittige Barrierelage herum, angeordnet werden.

Die Erfindung beschreibt insbesondere den Aufbau geruchshernmender bis geruchsdichter zu- und/oder ableitender Schlauchstrukturen, wie sie z.B. im zu- oder ableitenden Schlauchsegment verbaut werden, und die im Patienten platzierte, stuhlaufnehmende Kopfeinheit mit einer außerhalb des Körpers angeordneten Beuteleinheit verbindet. Bei stuhlableitenden Systemen werden die entsprechenden Schlauchsegmente bevorzugt derartig dünnwandig ausgeführt, so dass sie bereits bei kleiner Krafteinwirkung von außen zu flachen, bandartigen Strukturen kollabieren und somit der Entstehung von druckbedingten Läsionen der Haut des Patienten vorbeugen, insbesondere wenn der Körperstamm oder die Extremitäten des Patienten dem Schlauchsegment passager aufliegen. In der bevorzugten Ausführung des Schlauches richtet sich dieser bei nachlassender äußerer Krafteinwirkung, zumindest partiell, spontan-elastisch auf. Ein entsprechendes Aufrichtungsverhalten kann in idealer Weise durch die Verwendung von PUR als Trägermaterial realisiert werden. Zur Verwendung kommen beispielsweise PUR- bzw. TPU Typen der Shore Härte 80A bis 95A und 55D bis 65D. Der Durchmesser derartig elastisch wirkender, mit einer PUR-Trägerlage ausgerüsteten Schlauchsegmente liegt beispielsweise bei stuhlableitenden Systemen im Bereich von 15 bis 30 mm, bevorzugt 20 bis 25 mm, Die Wandungsstärke der Schlauchkomponente liegt typischerweise im Bereich von 200 bis 400 $\mu$m. Die Stärke der Barrierelage, beispielsweise aus EVOH, beträgt etwa 5 bis 25 $\mu$m, bevorzugt 10 bis 15 $\mu$m.

Die spontane Aufrichtung bzw. Rundung des Querschnitts nach einer passageren Verformung des stuhlableitenden Schlauchsegments durch eine von außen auf den Schlauch wirkende Kraft kann durch seriell aufeinander folgende, ringförmige, konvex oder konkav gerichtete Ausstülpungen oder Einstülpungen der Schlauchwandung unterstützt werden. Bei den oben genannten PURbezogenen Materialhärten, Wandungsstärken und Schlauchdurchmessern sind solche zirkulären Erweiterungen oder Reduktionen des Schlauchmantels bevorzugt 3 bis 6 mm breit und weisen Auslenkungen des Schlauchdurchmessers von im Scheitel 1,0 bis 3 mm, bevorzugt 1,0 bis 2,0 mm auf. Die Auslenkungen sind in der bevorzugten Ausführung jeweils bogenförmig, konvex oder konkav gewölbt.

Bei alleiniger Verwendung eines PVC-basierten Trägermaterials in Kombination mit einer EVOH oder PVDC basierten Trennschicht, sind die im Rahmen der Erfindung bevorzugten, PUR-typischen, elastischen Aufrichtungssei-

genschaften des Schlauchmantels nicht oder nur in kleinem Umfang erreichbar. Bei einer anteiligen Verwendung von PVC, als beispielsweise Innenlage, kann die Fähigkeit zur elastischen Selbstaufrichtung jedoch durch Aufnahme einer zusätzlichen PUR Lage in die Folienwandung integriert werden. Das verwendete PUR weist dann vorzugsweise eine relativ zum verwendeten PVC höhere Shore-Härte auf, im Bereich von beispielsweise Shore 90A oder auch Shore 55D. Bei einer angestrebten Gesamtwandungsstärke der Schlauchfolienwandung für einen stuhlableitenden Drainageschlauch, von 200 bis 400 μm, kann beispielhaft folgende Anordnung von Materiallagen kombiniert werden: außen - PUR 55D (50-100μm) mittig - EVOH oder PVDC (10-20μm), innen - PVC 60A-80A (140 bis 280μm). Eine nach innen, zum Drainagelumen ausgerichtete PVC-Lage ist im Rahmen der Erfindung konzeptionell von Vorteil, da der mit PVC erreichbare Barriereeffekt gegen Wasser den entsprechenden Barriereeffekt einer PUR-Lage gleicher Wandungsstärke übersteigt, Bei der Verwendung von EVOH als mittig verbaute Barriere-Lage ist ein Schutz vor Wassermolekülen von Vorteil, da die Barriere-Wirkung von EVOH durch eine Exposition mit Wasser reduziert wird.

PUR-basierte Materiallagen statten die im Rahmen der Erfindung beschriebenen, mehrlagigen, aus Schlauchfolien ausgeformten Komponenten der Kathetervorrichtung mit einer hohen mechanischen Stabilität und Belastbarkeit aus. Bereits dünnwandige, anteilige PUR-Lagen im Bereich von 10 bis 30 μm verleihen der jeweiligen Komponente, im Vergleich zu beispielsweise PVC, erheblich bessere Zug- und Reißfestigkeit, sowie Schnitt- und Punktionsresistenz.

Die für den Folienaufbau verwendeten Trägermaterialien PUR und PVC, sind mit gängigen Lösungsmitteln, wie beispielsweise Cyclohexanon oder Tetrahydrofuran verklebbar, was für die einfache Montage einer Kathetervorrichtung aus mehreren, separat hergestellten Baugruppen entscheidend ist. In den bevorzugten Ausführungen der hier beschriebenen, geruchsdicht optimierten Kathetervorrichtung, wird. das stuhlableitende Schlauchsegment zwischen einer distalen, im Rektum platzierten, vollständig PUR-basierten Kopfeinheit und einem proximalen, extrakorporalen Konnektorelement aus beispielsweise PUR, ABS oder PVC, jeweils durch Lösungsmittelklebungen verbunden.

Neben EVOH oder PVDC können, als weniger wirksame Geruchsbarriere, auch anteilige Lagen von Polyamid (PA) oder Pebax (TPE-A Polyamid), in eine erfindungsgemäße, mehrlagige Folie verbaut werden. Die Geruchsbarriere-Eigenschaften von PA erreichen nicht die Effizienz von EVOH oder PVDC, sind jedoch insbesondere bei kürzeren Anwendungszeiten einer erfindungsgemäßen Kathetervorrichtung vorteilhaft nutzbar.

Besonders vorteilhaft sind Kombinationen aus EVOH und PA, da sich beide Materialien insbesondere bei Koextrusionsverfahren gut und in der Regel ohne eine verbindende, adhäsionsunterstützende, sogenannte Tie-Layer-Lage verbinden lassen.

Ferner bieten sich Lagenkombinationen aus PA und PUR an, die ebenfalls die Option auf Koextrusion ohne haftungsvermittelnden Tie-Layer bieten. Durch Einsatz eines entsprechenden Haftungsvermittlers als Zwischenlage ist wiederum eine Verbindung von PA mit PE-basierten Lagen möglich.

Die für die Herstellung der Katheterkomponenten eingesetzten, mehrlagig extrudierten Schlauchfolienrohlinge lassen sich in einem nachfolgenden Herstellungsschritt, beispielsweise durch ein hot-molditig Verfahren, thermisch umformen, wobei der unbearbeitete Schlauchrahling, durch Beaufschlagung mit Druckluft, in eine erhitze Formkavität expandiert, und dort durch anschließende Kühlung des Formwerkzeuges in seiner Form fixiert wird. Neben einer derartigen Umformung des extra-korporalen, stuhlableitenden Schlauchsegmentes zu einem beispielsweise mit lumen-stabilisierenden Erweiterungen oder Einziehungen versehenen Schlauchmantel, umfasst die Erfindung auch Ausführungsformen, bei denen neben der Ausformung des stuhlableitenden Schlauchsegmentes gleichzeitig eine Ausformung der intra-korporalen Schaftschlauchkomponente sowie optional auch der intra-korporalen Ballonkomponente der Vorrichtung erfolgt. Die Erfindung beschreibt Ausführungsformen der Kathetervorrichtung, bei denen sowohl der gesamte intra- als auch der gesamte extra-korporale Anteil des Katheters aus einem einzigen, strukturell durchgängigen Folienschlauchrohling ausgeformt wird.

Die beschriebenen Folienrohlinge können durch direkte mehrlagige Extrusion zum Schlauch hergestellt werden, optional aber auch als Flachfolie zum Folienschlauch weiterverarbeitet werden. Es sind ferner mehrlagige Tauchverfahren denkbar, wobei die jeweiligen Materiallagen sukzessive von innen nach außen aufgetragen werden.

Die baulichen Merkmale der Erfindung werden im Detail in den folgenden Abbildungen erklärend dargestellt. Hierbei zeigt:

Fig. 1      eine beispielhafte Kathetervorrichtung, baulich ausgeführt für die kontinuierliche, trans-anale Ableitung von Stuhl, bestehend aus einer trans-anal und extra-korporal stuhiableitenden Schlauchkomponente und einer rektal platzierten Kopfeinheit mit einem Haltebailon,

Fig. 2      eine Drainage der Bauart nach Fig. 1, wobei sowohl der stuhlaufnehmende intra-korporale als auch der stuhlableitende extra-korporale Anteil der Schlauchkomponente aus mehrlagigem Folienmaterial hergestellt ist, und wobei die Folie mindestens eine geruchsdichte Barriere-Lage integriert,

Fig. 3      eine besondere Ausführung einer Drainage-Kopfeinheit, die eine hantel- oder auch pilzförmige Gestalt aufweist, wobei sich der rektal ankernde Ballonanteil in einen in den Analkanal oder durch diesen hindurch reichenden Ballonanteil fortsetzt,

Fig. 4a      einen spezifisch ausgeformten Schlauchrohling mit einer sphärischen oder diskoidal-ringartigen endständigen Erweiterung, für die Herstellung einer besonders einfach aufgebauten, geruchsreduzierenden Kathetervorrichtung,

Fig. 4b      einen aus dem in Fig. 4a abgebildeten Rohling hergestellten Bautyp einer Stuhldrainage, wobei die beiden Schlauchenden in sich rückgestülpt werden und somit ein toroidal-ringartiges, von außerhalb des Körpers befüllbares Ballonkompartiment erzeugen,

Fig. 4c      einen an Fig. 4a angelehnten, ausgeformten Schlauchrohling, mit einem sich der ausgeformten endständigen Erweiterung unmittelbar lateral anschließen Segment, mit einem wellig ausgeformten, das Schlauchlumen stabilisierenden Profil,

Fig. 4d      einen aus dem in Fig. 4c abgebildeten Rohling hergestellten Bautyp einer Stuhldrainage, wobei die beiden Schlauchenden in sich rückgestülpt werden, und das wellig ausgeformte Segment im Inneren des ringartigen, befüllbaren Kompartimentes zu liegen kommt und dort stabilisierend auf das stuhlaufnehmende Lumen der Drainage wirkt,

Fig. 4e      einen weiteren Formungsrohling, welcher zum einen eine hantelförmige, ballonartige Erweiterung, und zum anderen, ein sich der Erweiterung lateral anschließendes, welliges Profil aufweist, welches bei der Platzierung im Körper in den Analkanal hinein oder durch diesen hindurchreicht,

Fig. 4f      einen aus dem in Fig. 4e abgebildeten Rohling hergestellten Bautyp einer Stuhldrainage, wobei die beiden Schlauchenden in sich rückgestülpt werden, und das wellig ausgeformte Segment im inneren des hantelförmigen Ballonkompartiments zu liegen kommt und dort lumen-stabilisierend auf das Drainagelumen wirkt,

Fig. 5      einen beispielhaften, schematischen Wandungsaufbau einer geruchsreduzierenden, mit einer geruchsdichten Barriere-Lage aus EVOH oder PVDC ausgestatteten, mehrlagig aufgebauten Schlauchbzw. Ballonfolie,

Fig. 6      einen Fig. 5 entsprechenden, mehrlagigen Wandungsaufbau, wobei die geruchsreduzierende Barriere-Lage aus Polyamid (PA) oder PAverwandtem Pebax besteht,

Fig. 7      einen mehrlagigen Wandungsaufbau, wobei eine mittig angeordnete Barriere-Lage beidseits von PUR-Lagen eingeschlossen wird,

Fig. 8      einen mehrlagigen Wandungsaufbau, wobei eine mittig angeordnete Barriere-Lage aus EVOH jeweils nach außen hin durch eine Abfolge von PA, Tie-Layer und einer oder mehreren LDPE-Lagen ergänzt wird,

Fig. 9      einen konzeptionell zweilagigen Wandungsaufbau einer geruchsreduzierenden Ballon- oder Schlauchfolie, wobei die Barriere-Lage aus PVDC oder aus EVOH besteht und sowohl mit einer tragenden Lage aus PUR, PVC oder PA bzw. Pebax kombiniert sein kann;

Fig. 10a bis 10j      besondere Ausführungsformen erfindungsgemäßer Drainage-Kopfeinheiten, jeweils in einem Längsschnitt; sowie

Fig. 10k      einen Schnitt durch die Fig. 10h entlang der Linie XK - XK

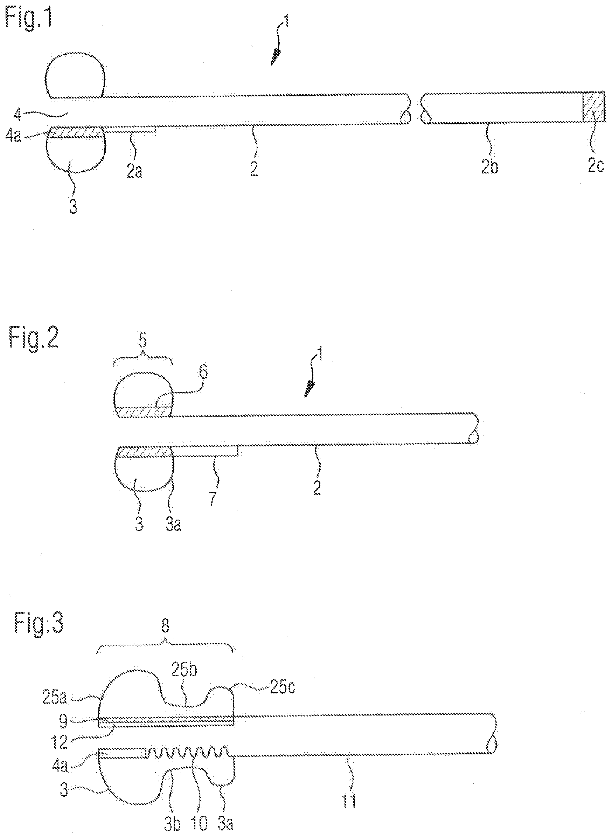

Fig 1 zeigt im schematischen Längsschnitt den Aufbau einer erfindungsgemäßen, katheterartigen Vorrichtung 1, welche in der abgebildeten Ausführung konzeptionell für die kontinuierliche Ableitung von Stuhl aus dem Rektum eines Patienten in einen extrakorporalen Sammelbehälter geeignet ist. Der Aufbau entspricht im Wesentlichen dem Bautyp herkömmlicher, silikon-basierter Stuhldrainagen.

Die Kathetervorrichtung besteht aus einem schlauchartigen Segment 2, dem am distalen, rektal positionierten Ende ein ankernd (retinierend) wirkender Ballonkörper 3 aufsitzt. In einer besonders einfachen Ausführung dieser Bauweise verfügt der toroidal-ringförmige Ballonkörper über keinerlei Versteifung, die die stuhlaufnehmende Mündung 4 der Vorrichtung für die Aufnahme und Ableitung von Darminhalt offenhält. Während bei silikonbasierten Drainagen die Öffnung der Mündung durch eine allseitige, radial nach außen gerichtete, elastische Expansion des torusförmigen Ballonkörpers sichergestellt wird, wobei nicht nur der äußere Durchmesser des Ballontorus zunimmt, sondern sich auch der Durchmesser der stuhlaufnehmenden, zentralen Öffnung weitet und diese somit stabilisiert, ist ein derartiger, lumenerhaltender bzw. -stabilisierender Effekt bei nicht oder nur wenig elastischen Materialien nicht gegeben. Bei der Beaufschlagung des Ballons mit Fülldruck kommt es hier zur einem, zum Ballonzentrum hin gerichteten Kollaps des den Ballon tragenden Schlauchlumens und somit zum Verschluss der Öffnung. Die vorliegende Erfindung begegnet dieser nach innen wirkenden Kraft, durch eine nach außen wirkende, elastische Selbstaufrichtung des ballontragenden Schlauchsegmentes.

Im Gegensatz zu relativ dickwandigen, sich elastisch auf ein zu erreichendes Arbeitsmaß aufdehnenden Ballonkomponenten aus Silikon, ermöglichen nicht- oder nur gering volumendehnbare Materialien, wie beispielsweise thermoplastische Polyurethane (TPU) im Härtebereich von Shore 80A bis 95A und 55D bis 65D, zum einen die vollständige Ausformung des Ballonkorpus auf sein für die Funktion erforderliches Arbeitsmaß bei der Herstellung, zum anderen sehr dünne Wandstärken im niedrigen Mikrometerbereich, von etwa 15 bis 35 μm. Die Verwendung von TPU ermöglicht somit die Herstellung besonders dünnwandiger, komplex ausgeformter Ballongeometrien, die bedingt durch die niedrige Material-Compliance (Volumen-Dehnbarkeit), trotz niedrigster Wandstärken, auch bei hohen, in situ auf den Ballon einwirkenden Kräften, verlässliche Formstabilität. Da eine elastische Aufdehnung der Ballonwandung nicht erforderlich ist, können die für die Funktion im Körper erforderlichen Fülldrucke relativ niedrig sein und sich beispielsweise im Falle der rektalen Stuhlableitung dem im Rektum herrschenden physiologischen Organdruck nähern. Diese Option bietet sich insbesondere bei einer inkompletten, schlaff spannungslosen Befüllung des Ballons.

Andere Bauarten silikonbasierter Stuhlableitungssysteme weisen im Mündungsbereich der Drainage zylindrisch geformte, sich elastisch verformende und aufrichtende, verstärkende hülsenartige Komponenten 4a auf, deren elastische Fähigkeit zur kreisrunden Aufrichtung den in situ, im Ballonkörper herrschenden Fülldruck bis zu einem bestimmten, den radialen Kollaps der verstärkenden Komponente verursachenden Druckwert überwiegt

Als weitere bauliche Variation silikonbasierter Stuhldrainagen sind Ausführungen bekannt, bei denen sich unmittelbar proximal zum intra-rektalen Halteballon, in trans-analer Positionierung, ein stuhlableitendes Schlauchsegment 2a anschließt, das aus einem besonders dünnwandigen und/oder auch besonders weichen Silikon besteht, wodurch insbesondere die mechanische Irritation der Schleimhaut des Analkanals durch den sich relativ zum Anus bewegenden und scheuernd wirkenden Schlauchmantel vermindert wird, Diese bauliche Anpassung des trans-analen Schlauchsegmentes begünstigt jedoch im Anwendungsverlauf axiale Verwindungen des Schlauches, die zum partiellen oder vollständigen Verschluss des ableitenden Lumens führen können.

Aus Silikon gefertigte Stuhldrainagen werden zum Teil mit zusätzlichen, die Geruchsfreisetzung hemmenden und/oder geruchsbindenden Materiallagen ausgestattet. Das den Stuhl innerhalb des Rektums aufnehmende und durch den Analkanal leitende Segment der Vorrichtung, wird, bedingt durch die eine der Regel ausgeprägte Steifigkeit der verwendeten Barrierematerialien, wie beispielsweise Parylene, typischerweise nicht mit derartigen geruchsreduzierenden Materiallagen oder Materialmodifikationen versehen.

Das proximale Schlauchsegment 2b geht am freien Ende des stuhlableitenden Katheters in ein Konnektorelement 2c über, welches den Anschluss bzw. den Wechsel eines Sammelbeutels erlaubt.

Fig. 2 zeigt eine erfindungsgemäße Ausführung einer stuhiableitenden Kathetervorrichtung 1, die sich konzeptionell an den zuvor beschriebenen, konventionellen Bautyp anlehnt. Die den Stuhl im Rektum aufnehmende und die Drainage dort verankernde, toroidal ringförmig ausgeformte Kopfeinheit 5 weist im Inneren des Ballonkompartiments 3 einen verformbaren, sich spontan-elastisch aufrichtenden, zylindrischen oder schlauchförmigen Innenkörper 6 auf. Der Ballonkörper 3a wird bereits bei der Herstellung auf sein vollständiges, für die rektale Retention erforderliches Maß ausgeformt. Der Ballonkörper wird nach innen durch einen abschließende Innenkörper 6 in befüllbarer Weise zum Kompartiment abgeschlossen. Er geht nach proximal in einen stuhlableitenden, trans-analen Schlauchanteil 7 über, der den Stuhl durch den Analkanal hindurchleitet.

im Sinne der angestrebten Reduktion der Geruchsfreisetzung, werden beim beschriebenen Bautyp sämtliche, dem Patientenstuhl exponierte Komponenten in einer mehrlagigen, eine geruchsdichte bzw. -reduzierende Barriere-Lage integrierenden Bauweise ausgeführt. Diese umfasst den Ballonkörper, den ballontragenden Innenkörper, als auch den sich extrakorporal an die Kopfeinheit anschließenden, stuhlableitenden Schlauch.

In baulicher Abwandlung dieser allseitig mit einer geruchsreduzierenden Barriere-Lage ausgestatteten Ausführung einer Kathetervorrichtung, kann auch lediglich der ableitende Schlauch 2 entsprechend geruchshemmend oder geruchsdicht ausgeführt werden, oder auch ergänzend, der den Ballon tragende Innenkörper 6.

Um eine permanente Dehnung des Bahnkörpers mit hohem Fülldruck zu vermeiden bzw. um die Option einer schlaffen, weitgehend druckpassiven, atraumatischen Befüllung des Ballons zu ermöglichen, und dabei gleichzeitig eine suffiziente Öffnung der Drainage-Mündung zu gewährleisten, wird der den Ballon tragende Schlauchinnenkörper mit der Fähigkeit zur elastisch wirkenden, spontanen Aufrichtung versehen. Es ist funktionell vorteilhaft, wenn das sich dem ballontragenden Schlauchinnenkörper proximal anschließende, durch den Anus hindurchführende Schlauchsegment ebenfalls mit der Fähigkeit zur elastischen Verformung und Aufrichtung ausgestattet ist, wobei das trans-anale Schlauchsegment durch die anliegende Kraftwirkung des Sphinkters auf ein relativ kleineres Durchmessermaß kollabiert werden soll, und sich bei entsprechend nachlassender Kraftwirkung wieder, aus dem kollabierten Zustand in seinen ausgeformten Ausgangszustand aufrichtet. Das trans-anale Segment 7 der Vorrichtung passt sich somit dem jeweiligen Tonus bzw. Öffnungszustand des Schließmuskels an.

Es ist ferner von funktionellem Vorteil, wenn nicht nur der intrakorporale, sondern auch der extrakorporale, stuhlableitende Schlauchanteil der Vorrichtung über die Fähigkeit zur spontan-elastischen Aufrichtung verfügt, Im Bereich der extrakorporalen Stuhlableitung können somit lumen-verschließende, adhäsive Effekte der benetzten Schlauchinnenflächen reduziert oder vermeiden werden. Eine entsprechende, lumen-aufrichtende Wirkung wird durch den Einbau entsprechend elastischer Materiallagen in die Schläuchkomponente erreicht. Eine lumenaufrichtende Wirkung kann ferner durch zirkulär um den Umfang verlaufende, ringartige Erweiterungen oder Einziehungen des Schlauchmantels unterstützt werden.

Die lumen-aufrichtenden Eigenschaften des den Ballon tragenden Innenkörpers werden bevorzugt derart eingestellt, dass sich im außerhalb des Körpers, frei befüllten Zustand, bei einem Ballonfülldruck von 50 mbar keine nach innen gerichtete, radiale Invagination oder Einfaltung der Wandung des Innenkörpers einstellt.

Fig. 3 zeigt eine erfindungsgemäß ausgeführte, stuhlableitende Kathetervorrichtung, die über einen besonderen, für die kontinuierliche, trans-anale Platzierung und Dichtung konfigurierten Kopfteil 8 verfügt. Der Kopfteil integriert einen hantelförmigen, mittig verjüngten bzw. taillierten oder alternativ auch pilzförmigen Ballonkorpus 3, welcher einem, den Ballon tragenden innenkörper 9 aufsitzt, der mit der Fähigkeit zur elastisch wirkenden, radialen Faltung bzw. Invagination bzw. zum partiellen oder vollständigen Kollaps und/oder einer elastisch wirkenden axialen Stauchung bzw. Knickung und elastischer Wiederaufrichtung ausgestattet ist.

Der Ballonkorpus 3 weist eine mittige Verjüngung 25b auf, die innerhalb des Analkanals platziert wird, wobei eine distal endständige Erweiterung 25a der Hantel im Rektum und eine proximal endständige Erweiterung 25c unmittelbar prä-anal zu liegen kommt.

Hierdurch wird zum einen eine ungewünschte, permanent dilatierend wirkende Öffnung des analen Schließmuskels verhindert, und zum anderen kann perforierenden Verletzungen bei einer in das Rektum und Sigma gerichteten, forcierten Dislokation der Vorrichtung vorbeugt werden. Zur Stärkung der elastischen Selbstaufrichtung des Schaftschlauches, wird dieser bevorzugt mit einem speziellen welligen Profil 10 ausgestattet, wodurch die Wandstärke des Innenkörpers 9 reduziert werden und die Aufrichtungseigenschaften des Schlauches dennoch erhalten werden können. Die wellige Profilgebung (Korrugation) erlaubt es, annähernd folienartig dünne Wandungscharakteristika des Innenkörpers zu erreichen.

Der Ballonkorpus wird in der bevorzugten Ausführung durch Blasformung aus PUR hergestellt. Er wird bereits bei der Herstellung mit den für die trans-anale Positionierung erforderlichen Maßen und geometrischen Formcharakteristika versehen. Der den Ballon tragende, sich elastisch im Rektum und im Analkanal verformende und selbstaufrichtende Innenkörper 9 reicht in der bevorzugten Ausführung vollständig durch den Anus hindurch.

Drainagen mit entsprechenden Kopfeinheiten werden beispielsweise in WO 2013/026564 beschrieben. In Abwandlung dieses besonderen Bautyps einer hantelförmigen, trans-anal platzierten und über den Schließmuskel hinweg dichtenden, rektal stuhlaufnehmenden und trans-anal stuhlableitenden Kopfeinheit, kann der Ballonkorpus 3 auch eine vereinfachte Pilzform aufweisen, wobei der Ballon kein prä-anal positioniertes, sphärisch oder diskoid erweitertes Ballonsegment 3a aufweist, sondern das mittige, verjüngte Ballonsegment 3b ohne endständige Erweiterung durch den Anus hindurchreicht, die Öffnung des Anus überragt oder mit der äußeren Öffnung des Anus bündig abschließt. Alternativ zu einem wellig korrugierten, ring- oder wendelartigen Profil, kann die Fähigkeit des den Ballon tragenden Schaftschlauches zur elastischen Verformung und Aufrichtung auch durch entsprechend wirkende elastische Komponenten 12, beispielsweise durch separate, hülsenartige oder netzartige, mit dem Schaftschlauch flächig verbundene Komponenten, oder auch in die Schlauchoberfläche flächig integrierte, netzartige oder wendelartige Verstrebungen, oder auch durch flächig verbundene, gelartige oder schaumartige Materiallagen verstärkt bzw. in ihren Aufrichtungseigenschaften modifiziert werden.

Extrakorporal schließt sich dem Schlauchinnenkörper der Kopfeinheit ein zu- oder ableitendes Schlauchsegment 11 an.

In Anlehnung an die in Fig. 2 beschriebene Bauart können, über den stuhlableitenden Schlauch 11 hinaus, auch der Innenkörper 9 und/oder der Ballonkorpus 3 aus erfindungsgemäß geruchsdichtem Schlauchmaterial hergestellt werden.

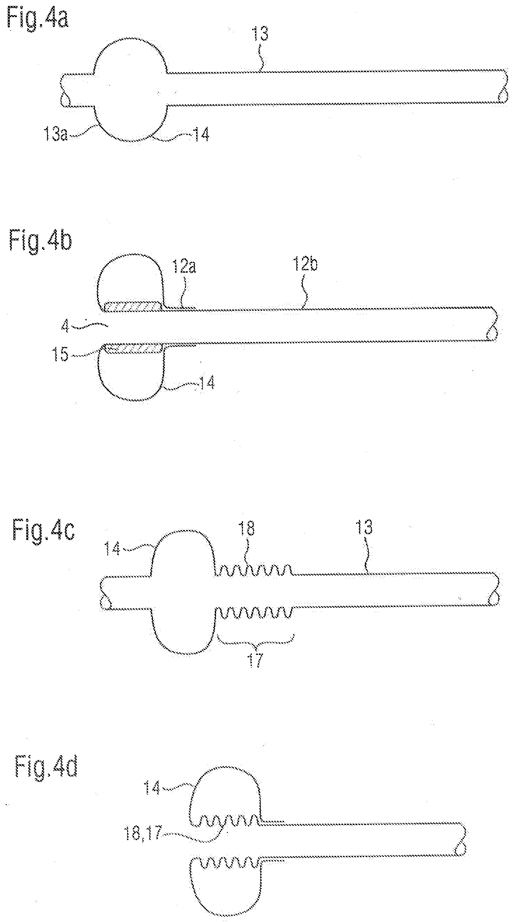

Fig. 4a beschreibt eine besonders einfach aufgebaute und wirtschaftlich vorteilhaft herstellbare Ausführungsform einer erfindungsgemäßen Kathetervorrichtung, deren Grundfigur vollständig aus einem einzigen, thermisch umgeformten Schlauchkörper 13 besteht. Das distale Ende 13a des Schlauchkörpers weist eine sphärische oder diskoide Erweiterung als Vorform für den Ballon 14 auf. Durch Rückstülpung der Erweiterung über den proximalen Anteil des Schlauchköpers entsteht ein torusförmiges, von extrakorporal befüllbares Kompartiment 14 in Form eines Ballons 3, 3, wie in Fig. 4b dargestellt.

Die zum Rektum weisende Mündung der Drainage wird durch ein, in das zentrale Lumen des torusartigen Ballons eingestecktes, elastisch verformbares und sich elastisch aufrichtendes Hülsenelement 15 offengehalten. Entsprechende einfach ausgeführte Bauformen stuhlableitender Systeme sind beispielsweise in WO 2009/144028 beschrieben. Das zentrale Lumen des torusartigen Ballons 3, 3a kann prinzipiell ohne ein zusätzliches, lumenstabilisierendes Hülsenelement ausgeführt werden. In diesem Falle weist der Schlauchkörper 13 selbst ausreichend starke, elastisch wirkende, das Drainagelumen erhaltende Selbstaufrichtungseigenschaften auf.

Fig. 4c zeigt einen Bautyp in Anlehnung an Fig. 4a, wobei die Fähigkeit zur lumenerhaltenden, elastischen Selbstaufrichtung des Schlauches 13 im vom befüllbaren Ballon eingeschlossenen Segment 17 durch ein in die Schlauchwandung eingeformtes ring- oder wendelartiges Profil 18 unterstützt wird. Bei der in Fig. 4d dargestellten Rückstülpung der endständig ausgeformten Erweiterung 14 des Schlauchkörpers kommt das so modifizierte, lumenerhaltende Schlauchprofil im Binnenraum 14 des Ballons zu liegen.

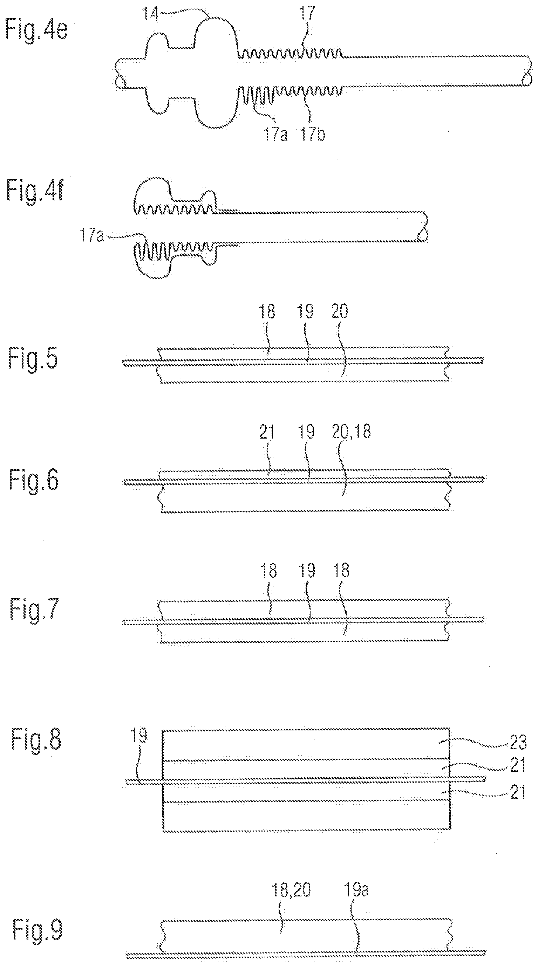

Fig. 4e zeigt eine entsprechende, wellig korrigierte Profilgebung des Schaftschlauches für trans-anal oder auch intra-anal positionierte bzw. dichtende Kopfeinheiten. Der durch eine wendelartige oder ringartige Profilgebung lumenstabilisierte Bereich 17 liegt nach der Rückstülpung der hantel- oder pilzförmigen Erweiterung 14 im inneren des befüllbaren Kompartimentes, wie in Fig. 4f dargestellt. Der mündungsnahe Anteil des Profils kann bei dieser baulichen Ausführung mit einer besonders eng gestellten und/oder hochamplitudigen, sich entsprechend stärker selbstaufrichtenden und weniger zum radialen Kollaps neigenden Korrugation 17a versehen werden, während der im Analkanal platzierte Anteil 17b, relativ zum Anteil 17a, eine weniger starke Aufrichtung gewährleistet, und somit die Strukturen des Analkanals entsprechend geringer und das anliegende Gewebe schonend, mit einer durchgängig wirkenden Kraft belastet.

Bei diesem Bautyp kann insbesondere im Bereich des rektal platzierten Segmentes der Kopfeinheit der Vorrichtung aus zusätzliche lumen-stabilisierende bzw. -aufrichtende Komponenten verzichtet werden, was einen entsprechenden Kostenvorteil bei der Herstellung der Kathetervorrichtung ermöglicht,

Die lumen-aufrichtenden Eigenschaften des den Ballon tragenden Innenkörpers werden durch eine entsprechende Kombination von Materialtypen, Materiallagenstärke und spezifischer Wellungscharakteristik bevorzugt derart eingestellt, dass sich im außerhalb des Körpers, frei befüllten Zustand, bei einem im Ballon herrschenden Fülldruck von 50 mbar keine nach innen gerichtete, radiale Einstülpung der Wandung des Innenkörpers einstellt.

Fig. 5 zeigt einen beispielhaften Wandungsaufbau der katheterartigen Vorrichtung, wobei die eingesetzten Materialtypen und deren spezifische, physikalische Eigenschaften zum einen im Sinne einer effizienten und körperverträglichen Funktion des Katheters, und zum anderen im Sinne einer effizienten Geruchsbarriere kombiniert werden. Die Gesamtwandungsstärke der für die Herstellung der Vorrichtung eingesetzten Folie, sowie die Materialhärte und die Stärke ihrer Einzellagen sind hierbei, in beispielhafter Ausführung, für die Anforderungen eines stuhlableitenden, dauerhaft im Rektum des Patienten verbleibenden Kathetersystems ausgelegt.

Im Bereich des intra- und extrakorporalen, stuhlableitenden Schlauches, optional auch im Bereich des Ballonkörpers, besteht das eingesetzte Folienmaterial aus einer Lage 18 von Polyurethan (PUR), gefolgt von einer mittig angeordneten Barriere-Lage 19 aus EVOH, Dieser Lage schließt sich eine Lage 30 aus PVC an.

Die Gesamtstärke der Schlauchwandung liegt bei 200 bis 400 $\mu$m, bevorzugt bei 250 bis 350 $\mu$m. Der PUR-Anteil 18 wird bevorzugt nach außen hin angeordnet und weist beispielsweise eine Lagenstärke von 200 $\mu$m auf, seine Materialhärte liegt im Bereich von Shore 80A bis 90A. Das PUR verleiht dem Schlauchkörper zum einen Zug- und Reißfestigkeit. Zum anderen stattet der PUR-Anteil die Schlauchfolie mit der Fähigkeit zur elastischen Verformung und Selbstaufrichtung aus. Die im Lagenverbund mittig angeordnete Barriere-Schicht 19 weist eine Wandungsstärke von ca. 15$\mu$m auf, sie besteht bevorzugt aus EVOH. Die Barriere-Schicht minimiert den Durchtritt von Wassermolekülen, Luftbestandteilen und geruchsintensiven Substanzen durch die Folienwandung. Die PVC-Lage 20 ist bevorzugt nach innen, zum Schlauchlumen hin gerichtet. Das PVC weist eine der Shore Härte 60A bis 80A, und eine Lagenstärke von ca. 100$\mu$m auf. Die PVC-Lage gewährleistet eine gewisse Barriere gegen Wassermoleküle, die die mittig angeordnete EVOH-Lage bis zu einem gewissen Grad von Wasser abgeschirmt, wodurch der Barriere-Effekt des EVOH nicht durch die Interaktion mit Wassermolekülen beeinträchtigt bzw. reduziert wird.

Um die in den voragusgehenden Figuren beschriebenen Bauformen der Vorrichtung herzustellen, bei denen sowohl der im Rektum retinierende Ballonanteil als auch das trans-anale und/oder das extra-korporale, stuhlableitende Schlauchsegment aus einem einzigen Schlauchrohling ausgeformt werden, schlägt die Erfindung eine angepasste Verteilung der oben genannten, anteiligen Materialstärken vor. Die PUR-Lage wird auf 280 $\mu$m, die EVOH oder PVDC-Lage wird auf 40 $\mu$m verstärkt. Zur Abschirmung des EVOH nach innen, wird eine PVC-Lage in reduzierter Stärke von ca. 80 $\mu$m verwendet. Diese dimensionale Anpassung der PUR-Lagenstärke ermöglicht die geometrisch stabile, symmetrische Blasformung der rektalen, ballonartigen Erweiterung aus dem in den Umformungsprozess eingesetzten, relativ zum Ballondurchmesser kleineren Ausgangsschlauch bzw. Rohling. Bei einem angenommenen Kalibersprung des Schlauchdurchmessers von 20-30 mm auf einen Ballondurchmesser von etwa 60-70 mm, verdünnt sich die EVOH-Lage im Verlauf einer Blasformung von 40$\mu$m auf etwa 10 bis 15 $\mu$m, was den Erhalt der Barrierefunktion im Ballon sicherstellt bzw. eine kritische Ausdünnung der Barrierelage ausschließt. In entsprechender Weise stellt der höhere PUR-Anteil an der Gesamtwandungsstärke des umzuformenden Rohschlauches sicher, dass der erweitert ausgeformte Ballonanteil eine ausreichende mechanische Belastbarkeit, insbesondere Formstabilität, Reißfestigkeit und Punktionsbeständigkeit aufweist.

Die beschriebene, höhere Wandungsstärke der äußeren PUR-Lage ist insbesondere für die simultane Blasformung des Ballonanteils und eines wellig korrugierten, das Drainagelumen im ballontragenden Bereich stabilisierenden Schlauchschlauches aus einem einzigen, durchgängigen Schlauchrohling vorteilhaft. Die Kombination der zuvor beschriebenen PUR-Typen und deren anteilige Lagenstärke, mit einem spezifisch gewellten, ringartig oder spiralig korrugierten Profil im ballontragenden bzw. vom Ballon eingeschlossenen Abschnitt des Schlauchschlauches, unterstützt dessen Fähigkeit zur spontanen Aufrichtung in die bei der Herstellung eingestellte Ausgangsform und trägt dazu bei, axial gerichtete Torsionen des Schlauches zu vermeiden.

Fig. 6 beschreibt einen weiteren beispielhaften Wandungsaufbau eines geruchsdichten bzw. die Freisetzung von Geruch hemmenden, folienartigen Schlauchmaterials, wobei die jeweilige, aus EVOH oder optional auch aus PVDC bestehende Barriereiage mit einer mechanisch stabilen Trägerlage 21 aus Polyamid (PA) oder aus dem Polyamid-verwandten Material, Pebax, kombiniert sein kann. In der Figur ist eine beispielhafte Kombination von Pebax außen, einer mittigen Lage aus EVOH und einer inneren Lage aus PVC 20 oder PUR 18 beschrieben. Bei einer Gesamtstärke, des in die thermische Umformung zum stuhlableitenden Segment eingesetzten Schlauchfolienrohlings von beispielsweise 300 μm, weist das Pebax eine Härte von Shore 35D bis 40D auf und hat eine Lagenstärke von 80 μm. Die mittige EVOH-Lage ist 10 bis 20 μm dick. Der innen liegende PVC- oder PUR-Anteil der Folie hat eine Stärke von 200μm.

Fig. 7 zeigt einen weiteren, mehrschichtigen Wandungsaufbau, der für die Herstellung der intra-korporalen Komponenten, umfassend den Ballonkörper, den ballontragenden Innenkörper und den extra-korporalen, stuhlableitende Schlauch, verwendeten Schlauchfolien-Rohlinge. Eine mittige Barriere-Lage 19 aus vorzugsweise EVOH, mit einer Wandungsstärke von 5 bis 20 μm, bevorzugt 10 bis 15 μm, wird zwischen zwei Trägerlagen 18 aus PUR mit Wandungsstärken von jeweils ca. 150 μm integriert. Die Materialhärte des PUR wird im Bereich von 80A bis 95A und 550 bis 65D eingestellt. Es können beim Aufbau des Schlauchrohlings auf der Außen- und Innenseite verschiedene PUR-Härten verwendet werden. Derartige Kombinationen von weicheren mit härteren PUR-Lagen können insbesondere dann vorteilhaft eingesetzt werden, wenn besondere elastische Aufrichtungseigenschaften, insbesondere des wellig korrugierten oder auch nicht korrugierten, ballontragenden Schaftschlauchsegmentes bzw. Innenkörpers erreicht werden sollen. Die Wandungsstärken der beiden Einzellagen können zur Erreichung optimaler elastischer Verformung und Wiederaufrichtung entsprechend über 150 μm angehoben (z.B. 250 μm eines 80A PUR-Typs, außen) oder unter 150 μm herabgesetzt werden (z.B. 50pm eines 60D PUR-Typs, innen). Ferner können zur Vermeidung PUR-typischer Klebrigkeit, auf der Oberfläche der jeweiligen, weicheren PUR-Trägerlagen, weitere, dünnwandige PUR-Lagen höherer Shore-Härte, von beispielsweise 5 bis 10 μm Lagenstärke aufgebracht werden, die insbesondere im Härtebereich von Shore 55D und darüber hinaus keinen derartigen klebrigen Aspekt ausbilden.

Fig. 8 beschreibt einen mehrlagigen Folienaufbau, wobei eine zentral angeordnete Barrierelage aus EVOH 19, beidseits flankierend, durch eine PA-Lage 21 ergänzt ist. Die beiden Materialien lassen sich in der Regel vorteilhaft einfach in einem Koextrusionsprozess kombinieren. Über eine Kleber-Lage (Tie-Layer) 22 werden dann eine oder mehrere Lagen eines LDPE-Materials 23 angebunden.

Fig. 9 beschreibt beispielhaft eine zweilagige Folienkombination, die eine EVOH oder PVDC basierte Barriere-Lage lediglich mit einer einzigen Trägerlage kombiniert, also auf eine sandwichartige Lagenanordnung, wie in den vorausgehenden Figuren beschrieben, verzichtet. Die Barriere-Lage 19 weist eine Wandungsstärke von 20 μm auf und wird bevorzugt als Außenfläche angeordnet. Die nach innen gerichtete Trägerlage 24 besteht bevorzugt aus PUR 18 oder PVC 20, und ist beispielhaft 200 bis 280 μm dick. Im Falle einer gleichzeitigen Ausformung sämtlicher Komponenten der Ballon- und Schaftschlauchkomponente der Kopfeinheit sowie des extra-korporalen, stuhlableitenden Schlauches aus einem einzigen eingesetzten Rohling, wird die Stärke der Barriere-Lage auf etwa 50μm, und die anteilige Lagenstärke des Trägers 18 oder 20 auf eine Stärke von 250 bis 300μm angehoben.

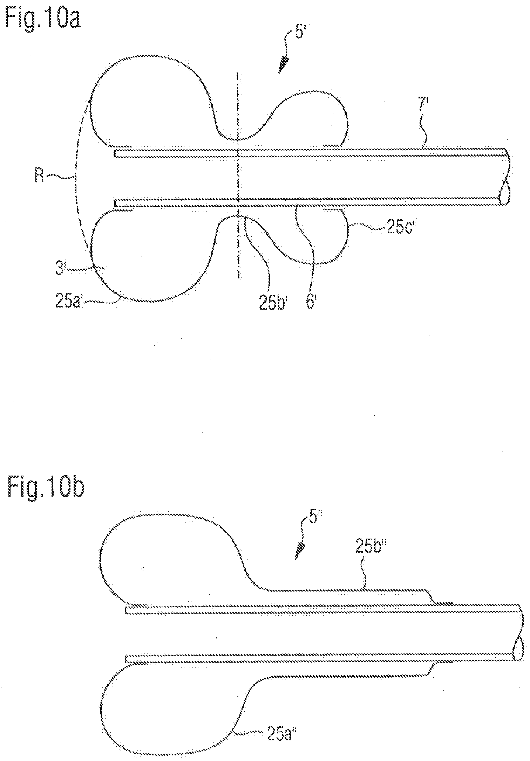

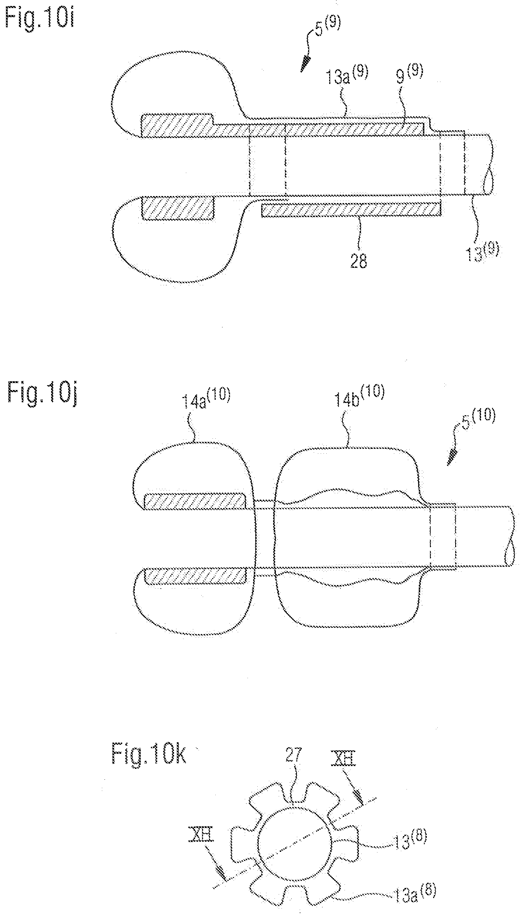

Fig. 10a zeigt eine Ausführung einer rektal stuhlaufnehmenden und trans-anal durchleitenden Kopfeinheit 5', umfassend eine hantelförrnig ausgeformte Ballonkomponente 3', welche einem elastisch verformbaren, sich spontan-elastisch in seine Ausgangsform aufrichtenden, zylindrischen oder schlauchförmigen Innenkörper 6' oder Schlauchanteil 7' aufsitzt. Die hantelförmige Ballonkomponente 3' wird bereits bei der Herstellung auf ihr vollständiges, für die rektale Retention erforderliches Maß ausgeformt. Sie weist eine mittige Verjüngung 25b' auf, die innerhalb des Analkanals platziert wird, wobei die distal endständige Erweiterung 25a' der Hantel im Rektum und die proximal endständige Erweiterung 25c' unmittelbar prä-anal zu liegen kommt. Die zum Rektum gerichtete Mündung 4' des schlauchförmigen Innenkörpers 6' überragt den distalen Radius R der auf dem Innenkörper 6' montierten Ballonkomponente 3' im befüllten Zustand nicht. Die von extrakorporal befüllbare Kopfeinheit 5' geht nach proximal in einen stuhlableitenden Schlauchanteil über, der die Kopfeinheit 5' mit einem extrakorporalen Behälter verbindet.

Fig. 10b zeigt eine von Fig. 10a abgeleitete Bauform einer Kopfeinheit 5'', wobei die Ballonkomponente 3'' eine pilzförmige Gestalt aufweist und auf eine proxinmal endständige, prä-anale Erweiterung verzichtet, sondern nur einen distalen, radial erweiterten Bereich 25a'' und einen proximal daran anschließenden, radial verjüngten Bereich 25b'' aufweist. Das proximale, trans-anal positionierte Segment 3b'' reicht optional in den Analkanal hinein, durch den Analkanal hindurch, oder ragt über die Öffnung des Anus hinaus.

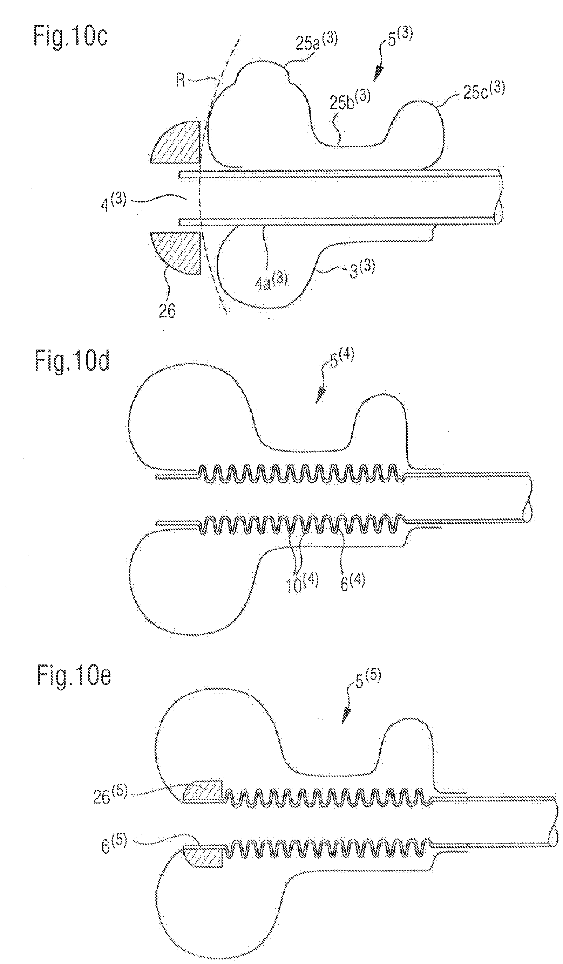

Fig. 10c enthält eine ebenfalls von den Figuren 10a und 10b abgeleitete Bauform einer Kopfeinheit 5$^{(3)}$ mit einer Ballonkomponente 3$^{(3)}$, bei der die zum Rektum gerichtete Mündung 4$^{(3)}$ des schlauchförmigen Innenkörpers 4a$^{(3)}$ den distalen Radius R der auf dem Innenkörper 4a$^{(3)}$ montierten Ballonkomponente 3$^{(3)}$ im befüllten Zustand überragt. Das frei in das Rektum hinein reichende Ende des Innenkörpers 4a$^{(3)}$ ist dabei vorzugsweise von einem besonders weich ausgeführten, kappenartigen oder olivenartigen Element 26 in nach lateral und frontal schützender Weise eingefasst,

Fig. 10d offenbart eine wiederum abgewandelte Bauform einer erfindungsgemäßen Kopfeinheit 5$^{(4)}$, wobei der den hantel- oder pilzförmigen Ballon 3$^{(4)}$ tragende Innenkörper 6$^{(4)}$ ein welliges, ring- oder wendelartig ausgeformtes Profil 10$^{(4)}$ aufweist, welches sowohl im rektalen als auch im trans-analen Segment, als auch in beiden Segmenten der Kopfeinheit 5$^{(4)}$ integriert sein kann und so die elastischen Verformungs- und Aufrichtungseigenschaften des ballontragenden Innenkörpers 6$^{(4)}$ vorteilhaft modifiziert, indem die radiale Verformung und Aufrichtung in einer beschleunigten, besonders prompten Art und Weise erfolgt. Die baulich geometrische Ausführung welligen Profils 10$^{(4)}$ kann dabei segmentspezifisch variiert werden. Beispielsweise kann das Wellungsprofil 10$^{(4)}$ im Bereich des rektalen positionierten Segments der Kopfeinheit 5$^{(4)}$ derart ausgeformt sein, dass die lumenerhaltende bzw.-aufrichtende Wirkung der Wellung 10$^{(4)}$ die entsprechende Wirkung im trans-analen Segment der Kopfeinheit 5$^{(4)}$ überwiegt, was im rektalen Segment dazu beiträgt, den Mündungsbereich zum Rektum zu stabilisieren und im trans-analen Segment die Verformungs- und Aufrichtungseigenschaften derart einstellt, dass sich der Schaftinnenkörper 6$^{(4)}$ bei normalem Schließmuskeltonus radial einstülpt und sich bei nachlassendem Tonus prompt elastisch in den sich öffnenden Analkanal hinein entwickelt.

Fig. 10e zeigt eine aus Fig. 10d abgeleitete Bauform einer erfindungsgemäßen Kopfeinheit 5$^{(5)}$, bei der das rektale Segment des Innenkörpers 6$^{(5)}$ durch ein lumenstabilisierendes, ring- oder hülsenartiges Element 26$^{(5)}$ erfindungsgemäßen Kopfeinheit 5$^{(4)}$ ergänzt wird, welches flächig mit der innen- oder Außenfläche des rektalen Segmentes des Innenrohres 6$^{(5)}$ verbinden ist.

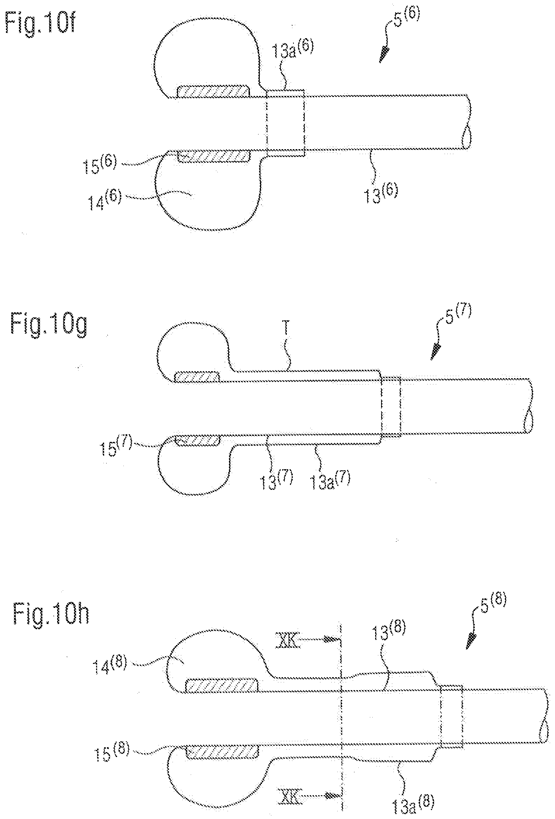

Fig. 10f zeigt eine besonders einfache Bauform einer stuhlableitenden Kopfeinheit 5$^{(6)}$, wobei das eine verjüngte Ende 13$^{(6)}$ einer sphärisch oder diskoid ausgeformten Ballonschlauchkomponente durch das andere verjüngte Ende 13a$^{(6)}$ hindurchgestülpt ist und die beiden Enden 13$^{(6)}$, 13a$^{(6)}$ miteinander flächig, dicht schließend zum befülbaren Kompartiment 14$^{(6)}$ verbunden sind, welches somit einen in einem Körperbinnenraum platzierbaren, retinierend wirkenden Ballonanker ausbildet. Zur Stabilisierung der stuhlaufnehmenden Mündung ist das rektale Segment mit einem ring- oder hülsenartigen Element 15$^{(6)}$ ausgestattet.

Fig. 10g gibt eine aus Fig. 10f abgeleitete Bauform einer stuhlableitenden Kopfeinheit 5$^{(7)}$ wieder, bei welcher die beiden verjüngten Ballonenden 13$^{(7)}$ und 13a$^{(7)}$ in den Analkanal hinein, oder auch durch durch ihn hindurchgeführt sind. Im trans-analen Segment T liegen die beiden Ballonenden 13$^{(7)}$, 13a$^{(7)}$ dann in konzentrischer Anordnung. Im trans-analen Segment T ist kein weiteres, lumenaufrichtendes Element verbaut. Auch hier kann zur Stabilisierung der stuhlaufnehmenden Mündung das rektale Segment mit einem ring- oder hülsenartigen Element 15$^{(7)}$ versehen sein.

Fig. 10h zeigt eine von Fig. 10g abgeleitete Ausführungsform einer stuhlableitenden Kopfeinheit 5$^{(8)}$, bei der die beiden konzentrischen, trans-anal positionierten Ballonenden 13$^{(8)}$, 13a$^{(8)}$ durch gleichmäßig über den Umfang des Segmentes verteilte, längsverlaufende, stegartige Schweißungen 27 miteinander verbunden sind, Kommt es zu einer Verschiebung von Volumen aus dem rektalen Segment des befüllbaren Kompartimentes 14$^{(8)}$ in das trans-anale Segment, richtet sich dieses luftmatratzenartig auf und gibt somit das zu- und oder ableitende Lumen frei Wiederum kann zur Stabilisierung der stuhlaufnehmenden Mündung das rektale Segment mit einem ring- oder hülsenartigen Element 15$^{(8)}$ versehen sein.

Fig. 10i zeigt eine eine stuhlableitende Kopfeinheit 5$^{(9)}$ als bauliche Ergänzung zu Fig. 10g, bei der im trans-analen Segment ein oder mehrere, elastisch verformbare, lumenstabilisierende, hülsen- oder ringartige Komponenten 9$^{(9)}$, 28 auf der Außenfläche und/oder Innenfläche der inneren der beiden konzentrischen Lagen 13$^{(9)}$, 13a$^{(9)}$ verbaut sind, die das trans-anale Segment zum einen in den sich öffenen Schließmuskel hinein aufrichten, zum anderen dem im befüllbaren Kompartiment herrschenden Fülldruck wiederstehen und einem nicht erwünschten Kollaps des zu- und/oder ableitenden Lumens vorbeugen.

Fig. 10j stellt einen weitere Bauform einer Kopfeinheit 5$^{(10)}$ dar, bei der das rektale und das trans-anale Segment strukturell separiert sind bzw. sich zwei separat befüllbare Kompartimente 14a$^{(10)}$ und 14b$^{(10)}$ seriell aneinander reihen, wobei das trans-anale Segment bevorzugt aus zwei konzentrischen, endständig zum geschlossenen, befüllbaren Raum verschweißten Schlauchlagen aufgebaut ist, und wobei das rektal platzierte Kompartiment 14a$^{(10)}$ eine die Kopfeinheit 5$^{(10)}$ ankernde, und das trans-anale Segment eine den Analkanal dichtende und gleichzeitg das zu- und/oder ableitende Lumen verschließende Funktion haben. Diese besondere Bauform gestattet bei passagerer Befüllung des trans-analen Kompartiments eine besonders kräftige, effiziente trans-anale Dichtungsleistung, Wird das Kompartiment evakuiert liegen die beiden Schlauchlagen einander dicht an, und geben das Lumen der Kopfeinheit vollständig frei.

Bezugszeichenliste

| | | | |
|---|---|---|---|
| 1 | katheterartige Vorrichtung | 18 | Profil, PUR-Lage |
| 2 | schlauchartiges Segment | 19 | Barriere-Lage |
| 2a | Schlauchsegment, | 20 | PVC-Lage |
| 2b | Schlauchsegment | 21 | PA-Lage |
| 2c | Konnektorelement | 22 | Kleber-Lage |
| 3 | Ballonkörper, -segment | 23 | LPDE-Lage |
| 3a | Ballonsegment | 24 | Träger-Lage |
| 3b | Ballonsegment | 25a | distale Erweiterung |
| 4 | Mündung | 25b | mittige Verjüngung |
| 4a | hülsenartige Komponente | 25c | proximale Erweiterung |
| 5 | Kopfeinheit | 26 | olivenartiges Element |
| 6 | Innenkörper | 27 | Schweißung |
| 7 | Schlauchanteil, -segment | 28 | Komponente |
| 8 | Kopfteil | | |
| 9 | Innenkörper | | |
| 10 | welliges Profil | | |
| 11 | Schlauchsegment | | |
| 12 | Komponente | | |
| 13 | Schlauchkörper | | |
| 13a | distales Ende | | |
| 14 | Erweiterung, Kompartiment | | |
| 15 | Hülsenelement | | |
| 17 | Segment | | |
| 17a | Korrugation | | |
| 17b | Anteil | | |

**Patentansprüche**

1. Katheterartige Vorrichtung (1) für die medizinische Zuleitung und/oder Ableitung von besonders geruchsintensiven, besonders löslichen oder sonstig migrationsfähigen Substanzen, umfassend

   - eine die in einem Organ oder in einem Körperbinnenraum platzierte Vorrichtung (1) ankernd retinierende Ballonkomponente (3,3a),
   - eine sich an die Ballonkomponente (3,3a) anschließende, transluminale, zum Organ oder zum Binnenraum führende zu- und/oder ableitende Schlaucheinheit (2a), sowie
   - einen sich daran, nach extrakorporal anschließenden, schlauchartigen Anteil (2),

   **dadurch gekennzeichnet, dass** wenigstens der extrakorporale, schlauchartige Anteil (2) aus einer mehrlagigen Folie gebildet wird, wobei die mehrlagige Folie wenigstens eine Barriere-Lage (19) aus einem geruchsdichtenden Barrierematerial umfasst sowie wenigstens eine Träger-Lage (24) aus einem anderen, mechanisch stabilen Material, wobei die Wandstärke der Träger-Lage (24) größer ist als die Wandstärke der Barriere-Lage (19), wobei eine oder mehrere Lagen des mehrlagigen Folienmaterials miteinander koextrudiert sind und wobei die (Gesamt-) Dicke $d_T$ der Träger-Lage (24) oder aller Träger-Lagen (24) gleich oder größer ist als das Fünffache der (Gesamt-) Dicke $d_B$ der Barriere-Lage (19) oder aller Barriere-Lagen (19):

$$d_T \geq 5 * d_B.$$

2. Vorrichtung (1) nach Anspruch 1, **dadurch gekennzeichnet, dass** neben dem zu- und/oder ableitenden, extrakorporalen schlauchartigen Anteil (2) auch der den Ballon (3,3a) tragende Innenkörper (6) und/oder der Ballonkorpus (3,3a) aus einem mehrlagigen, wenigstens eine geruchsdichtende Barriere-Lage (19) enthaltenden Folienmaterial hergestellt ist.

3. Vorrichtung (1) nach Anspruch 1 oder 2, **dadurch gekennzeichnet, dass** die Barriere-Lage (19) aus Ethylen-Vinylalkohol-Copolymer (EVOH) und/oder Polyvinylidenchlorid (PVDC) besteht.

4. Vorrichtung (1) nach einem der vorhergehenden Ansprüche, **dadurch gekennzeichnet, dass** neben der extrakorporalen Schlaucheinheit (2), auch der den Ballon (3,3a) tragende Schlauchinnenkörper (6) und optional auch die Ballonkomponente (3,3a) aus einem mehrlagigen, geruchsdichten, mit anteiligen Barriere-Lagen (19) aus EVOH und/oder PVDC ausgestatteten Folienmaterial besteht, wobei die entsprechenden Anteile der Vorrichtung (1) aus einem einzigen Schlauchrohling hergestellt werden.

5. Vorrichtung (1) nach einem der vorhergehenden Ansprüche, **dadurch gekennzeichnet, dass** die Schaftschlauchkomponente der Kopfeinheit (5,8) der Vorrichtung (1) derart mit einem spezifischen Profil ausgeformt und/oder mit zusätzlichen baulichen Komponenten derart verstärkt oder modifiziert ist, dass sich elastische Verformungs- und Aufrichtungseigenschaften einstellen, die gewährleisten, dass der außerhalb des Körpers mit einem Ballonfülldruck von mindestens 50 mbar belastete Schaftschlauch keine radial nach innen gerichtete Verformungen des Schlauchprofils ausbildet.

6. Vorrichtung (1) nach einem der vorhergehenden Ansprüche, **dadurch gekennzeichnet, dass** das mehrlagige Folienmaterial aus einer sandwichartigen Kombination besteht, wobei wenigstens eine mittige Barriere-Lage (19) an beiden Seiten von wenigstens je einer Träger-Lage (24) umgeben ist.

7. Vorrichtung (1) nach einem der vorhergehenden Ansprüche, **dadurch gekennzeichnet, dass** die geruchsdichtende Barriere-Lage (19), bestehend aus EVOH und/oder PVDC, zwischen einer Lage (18) aus Polyurethan (PUR) und einer Lage (20) aus Polyvinylchlorid (PVC) in den Lagenverbund der Folienrohlinge eingebettet ist.

8. Vorrichtung (1) nach einem der vorhergehenden Ansprüche, **dadurch gekennzeichnet, dass** die geruchsdichtende Barriere-Lage (19), bestehend aus EVOH und/oder PVDC, zwischen einer Lage (21) aus Polyamid (PA) und einer Lage (20) aus PVC in den Lagenverbund der Folienrohlinge eingebettet ist.

9. Vorrichtung (1) nach einem der vorhergehenden Ansprüche, **dadurch gekennzeichnet, dass** die geruchsdichtende Barriere-Lage (19), bestehend aus EVOH und/oder PVDC,

   a) zwischen einer äußeren und einer inneren Lage (18) aus PUR eingebettet ist, und/oder
   b) mit einer einzigen Trägerlage (24) aus PUR, PVC oder auch PA oder einem thermoplastischen Polyamidelastomer (TPE-A) kombiniert ist.

10. Vorrichtung (1) nach einem der vorhergehenden Ansprüche, **dadurch gekennzeichnet, dass** die in die jeweiligen Folienkombinationen verbauten PUR-Lagen (18) aus Polyurethanen der Härten nach Shore 80A bis 95A und 55D bis 65D bestehen.

11. Vorrichtung (1) nach einem der vorhergehenden Ansprüche, **dadurch gekennzeichnet, dass** die geruchshemmenden Barriere-Lagen (19), im Verbund mit den Träger-Lagen (24) jeweils Wandungsstärken von 5 bis 20 $\mu$m aufweisen.

12. Vorrichtung (1) nach einem der vorhergehenden Ansprüche, **dadurch gekennzeichnet, dass** eine oder mehrere Komponenten der katheterartigen Vorrichtung (1) miteinander verklebt sind, bspw. indem zwei beim Zusammenfügen aneinander liegende Oberflächenbereiche der zusammenzufügenden Komponenten ein Verkleben mit einem Lösungsmittel erlauben, insbesondere mit einem Lösungsmittel wie Cyclohexanon oder Tetrahydrofuran.

13. Vorrichtung (1) nach einem der vorhergehenden Ansprüche, **dadurch gekennzeichnet, dass** wenigstens eine Barriere-Lage (19), insbesondere eine Barriere-Lage (19) aus EVOH, mit einer benachbarten Lage (21) aus PA verbunden ist, insbesondere ohne eine verbindende, adhäsionsunterstützende Tie-Layer-Lage.

14. Vorrichtung (1) nach einem der vorhergehenden Ansprüche, **dadurch gekennzeichnet, dass** wenigstens eine Träger-Lage (24), insbesondere eine Träger-Lage (24) aus PUR, mit einer benachbarten Lage (21) aus PA verbunden ist, insbesondere ohne eine verbindende, adhäsionsunterstützende Tie-Layer-Lage.

**15.** Vorrichtung (1) nach einem der vorhergehenden Ansprüche, **dadurch gekennzeichnet, dass** eine Lage (21) aus PA durch Einsatz eines Hafvermittlers (22) als Zwischenlage mit einer PE-basierten Lage (23) verbunden ist.

**16.** Vorrichtung (1) nach einem der vorhergehenden Ansprüche, **dadurch gekennzeichnet, dass** die (Gesamt-) Dicke $d_T$ der Träger-Lage (24) oder aller Träger-Lagen (24) gleich oder größer ist als das Zehnfache der (Gesamt-) Dicke $d_B$ der Barriere-Lage (19) oder aller Barriere-Lagen (19):

$$d_T \geq 10 * d_B,$$

oder dass die (Gesamt-) Dicke $d_T$ der Träger-Lage (24) oder aller Träger-Lagen (24) gleich oder größer ist als das Zwanzigfache der (Gesamt-) Dicke $d_B$ der Barriere-Lage (19) aus EVOH oder PVDC oder aller Barriere-Lagen (19) aus EVOH und/oder PVDC:

$$d_T \geq 20 * d_B,$$

oder dass die (Gesamt-) Dicke $d_T$ der Träger-Lage (24) oder aller Träger-Lagen (24) gleich oder größer ist als das Vierzigfache der (Gesamt-) Dicke $d_B$ der Barriere-Lage (19) aus EVOH oder PVDC oder aller Barriere-Lagen (19) aus EVOH und/oder PVDC:

$$d_T \geq 40 * d_B.$$

**Claims**

**1.** A catheter-like device (1) for the medical delivery and/or discharge of substances that are particularly odor-intensive, particularly soluble, or otherwise capable of migration, comprising

- a balloon component (3, 3a) that anchors and retains a device (1) that is placed in an organ or in a body interior space,
- a delivering and/or discharging tube unit (2a) that adjoins the balloon component (3, 3a) and leads transluminally to the organ or to the interior space, and
- an extracorporeally adjoining tube-like portion (2),

**characterized in that** at least the extracorporeal tube-like portion (2) is formed from a multilayer film, the multilayer film including at least one barrier layer (19) made of an odor-proof barrier material and at least one carrier layer (24) made of some other, mechanically stable material, the wall thickness of the carrier layer (24) being greater than the wall thickness of the barrier layer (19), wherein one or more layers of the multilayer film material are coextruded with one another and wherein the (overall) thickness $d_T$ of the carrier layer (24) or of all carrier layers (24) is greater than or equal to five times the (overall) thickness $d_B$ of the barrier layer (19) or of all barrier layers (19):

$$d_T \geq 5 * d_B.$$

**2.** The device (1) according to Claim 1, **characterized in that** in addition to the delivering and/or discharging extracorporeal tube-like portion (2), the inner body (6) that carries the balloon (3, 3a) and/or the balloon body (3, 3a) is manufactured from a multilayer film material containing at least one odor-proof barrier layer (19).

**3.** The device (1) according to Claim 1 or 2, **characterized in that** the barrier layer (19) is made of ethylene-vinyl alcohol copolymer (EVOH) and/or polyvinylidene chloride (PVDC).

4. The device (1) according to one of the preceding claims, **characterized in that** in addition to the extracorporeal tube unit (2), the tube inner body (6) that carries the balloon (3, 3a) and optionally also the balloon component (3, 3a) is made of a multilayer, odor-proof film material that is provided with proportional barrier layers (19) made of EVOH and/or PVDC, the corresponding portions of the device (1) being manufactured from a single tube blank.

5. The device (1) according to one of the preceding claims, **characterized in that** the shaft tube component of the head unit (5, 8) of the device (1) is formed with a specific profile and/or reinforced or modified with additional structural components in such a way that elastic deformation and straightening properties result which ensure that the shaft tube, when subjected to a balloon filling pressure of at least 50 mbar outside the body, forms no radially inwardly directed deformations of the tube profile.

6. The device (1) according to one of the preceding claims, **characterized in that** the multilayer film material is made up of a sandwich-like combination, at least one central barrier layer (19) being enclosed on both sides by at least one carrier layer (24) each.

7. The device (1) according to one of the preceding claims, **characterized in that** the odor-proof barrier layer (19) made of EVOH and/or PVDC is embedded in the layer composite of the film blanks, between a layer (18) made of polyurethane (PUR) and a layer (20) made of polyvinyl chloride (PVC).

8. The device (1) according to one of the preceding claims, **characterized in that** the odor-proof barrier layer (19) made of EVOH and/or PVDC is embedded in the layer composite of the film blanks, between a layer (21) made of polyamide (PA) and a layer (20) made of PVC.

9. The device (1) according to one of the preceding claims, **characterized in that** the odor-proof barrier layer (19) made of EVOH and/or PVDC

a) is embedded between an outer layer and an inner layer (18) made of PUR, and/or
b) is combined with a single carrier layer (24) made of PUR, PVC, or also PA or a thermoplastic polyamide elastomer (TPE-A).

10. The device (1) according to one of the preceding claims, **characterized in that** the PUR layers (18) inserted into the particular film combinations are made of polyurethanes having Shore hardnesses of 80A to 95A and 55D to 65D.

11. The device (1) according to one of the preceding claims, **characterized in that** the odor-inhibiting barrier layers (19), in combination with the carrier layers (24), in each case have wall thicknesses of 5 to 20 $\mu$m.

12. The device (1) according to one of the preceding claims, **characterized in that** one or more components of the catheter-like device (1) are adhered to one another, for example by allowing two surface regions, adjacently situated for the joining, of the components to be joined to become adhered using a solvent, in particular using a solvent such as cyclohexanone or tetrahydrofuran.

13. The device (1) according to one of the preceding claims, **characterized in that** at least one barrier layer (19), in particular a barrier layer (19) made of EVOH, is joined to a neighboring layer (21) made of PA, in particular without a connecting, adhesion-assisting tie layer.

14. The device (1) according to one of the preceding claims, **characterized in that** at least one carrier layer (24), in particular a carrier layer (24) made of PUR, is joined to a neighboring layer (21) made of PA, in particular without a connecting, adhesion-assisting tie layer.

15. The device (1) according to one of the preceding claims, **characterized in that** a layer (21) made of PA is joined to a PE-based layer (23) by use of an adhesion promoter (22) as an intermediate layer.

16. The device (1) according to one of the preceding claims, chataracterized in that or that the (overall) thickness $d_T$ of the carrier layer (24) or of all carrier layers (24) is greater than or equal to ten times the (overall) thickness $d_B$ of the barrier layer(19) or of all barrier layers (19):

$$d_T \geq 10 * d_B,$$

or that the (overall) thickness $d_T$ of the carrier layer (24) or of all carrier layers (24) is greater than or equal to twenty times the (overall) thickness $d_B$ of the barrier layer (19) made of EVOH or PVDC or of all barrier layers (19) made of EVOH and/or PVDC:

$$d_T \geq 20 * d_B,$$

or that the (overall) thickness $d_T$ of the carrier layer (24) or of all carrier layers (24) is greater than or equal to forty times the (overall) thickness $d_B$ of the barrier layer (19) made of EVOH or PVDC or of all barrier layers (19) made of EVOH and/or PVDC:

$$d_T \geq 40 * d_B.$$

**Revendications**

1. Dispositif (1) de type cathéter pour l'administration médicale et/ou l'évacuation de substances particulièrement odorantes, particulièrement solubles ou tout autre substance susceptible de migrer, comportant

   - un composant de ballonnet (3, 3a) de retenue ancrant le dispositif (1) placé dans un organe ou dans un espace intérieur du corps,
   - une unité de tube (2a) d'administration et/ou d'évacuation translu-minale se raccordant au composant du ballonnet (3, 3a), menant à l'organe ou à l'espace intérieur, ainsi qu'
   - une partie en forme de tube (2) qui s'y raccorde de manière extracorporelle,

   **caractérisé en ce qu'**au moins la partie en forme de tube (2) extracorporelle est formée à partir d'un film multicouche, **en ce que** le film multicouche comporte au moins une couche barrière (19) constituée d'un matériau barrière anti-odeurs ainsi qu'au moins une couche support (24) constituée d'un autre matériau mécaniquement stable, **en ce que** l'épaisseur de paroi de la couche support (24) est supérieure à l'épaisseur de paroi de la couche barrière (19), **en ce qu'**une ou plusieurs couches du matériau de film multicouche sont coextrudées les unes avec les autres et **en ce que** l'épaisseur (totale) $d_T$ de la couche support (24) ou de toutes les couches supports (24) est égale ou supérieure à cinq fois l'épaisseur (totale) $d_B$ de la couche barrière (19) ou de toutes les couches barrières (19) :

$$d_T \geq 5 * d_B.$$

2. Dispositif (1) selon la revendication 1, **caractérisé en ce que**, outre la partie en forme de tube (2) extracorporelle d'administration et/ou d'évacuation, le corps intérieur (6) portant le ballonnet (3, 3a) et/ou le corps de ballonnet (3, 3a) est/sont également fabriqué(s) à partir d'un matériau de film multicouche, contenant au moins une couche barrière (19) anti-odeurs.

3. Dispositif (1) selon la revendication 1 ou 2, **caractérisé en ce que** la couche barrière (19) est constituée de copolymère d'éthylène-alcool vinylique (EVOH) et/ou de chlorure de polyvinylidène (PVDC).

4. Dispositif (1) selon l'une des revendications précédentes, **caractérisé en ce que**, outre l'unité de tube (2) extracorporelle, le corps intérieur de tube (6) portant le ballonnet (3, 3a) et en option également le composant de ballonnet (3, 3a) est constitué d'un matériau de film multicouche, anti-odeurs, doté d'une proportion de couches barrières (19) en EVOH et/ou PVDC, **en ce que** les parties correspondantes du dispositif (1) sont fabriquées à partir d'une seule ébauche de tube.

**5.** Dispositif (1) selon l'une des revendications précédentes, **caractérisé en ce que** le composant du tube tige de l'unité de tête (5, 8) du dispositif (1) est formé avec un profil spécifique et/ou renforcé ou modifié avec des composants de construction supplémentaires de telle sorte qu'il en résulte des propriétés de déformation et de redressement élastiques qui garantissent que le tube tige soumis à l'extérieur du corps à une pression de remplissage de ballonnet d'au moins 50 mbar ne forme pas de déformations du profil de tube orientées radialement vers l'intérieur.

**6.** Dispositif (1) selon l'une des revendications précédentes, **caractérisé en ce que** le matériau de film multicouche est constitué d'une combinaison de type sandwich, **en ce qu'**au moins une couche barrière (19) centrale est entourée des deux côtés par au moins une couche support (24).

**7.** Dispositif (1) selon l'une des revendications précédentes, **caractérisé en ce que** la couche barrière (19) anti-odeurs, constituée d'EVOH et/ou de PVDC, est insérée entre une couche (18) de polyuréthane (PUR) et une couche (20) de polychlorure de vinyle (PVC) dans le composite de couches des ébauches de film.

**8.** Dispositif (1) selon l'une des revendications précédentes, **caractérisé en ce que** la couche barrière (19) anti-odeurs, constituée d'EVOH et/ou de PVDC, est insérée entre une couche (21) de polyamide (PA) et une couche (20) de PVC dans le composite de couches des ébauches de film.

**9.** Dispositif (1) selon l'une des revendications précédentes, **caractérisé en ce que** la couche barrière (19) anti-odeurs, constituée d'EVOH et/ou de PVDC,

a) est insérée entre une couche extérieure et une couche intérieure (18) en PUR, et/ou
b) est combinée avec une seule couche support (24) en PUR, PVC,
ou aussi en PA ou en élastomère polyamide thermoplastique (TPE-A).

**10.** Dispositif (1) selon l'une des revendications précédentes, **caractérisé en ce que** les couches PUR (18) intégrées dans les combinaisons de films respectives sont constituées de polyuréthanes de dureté Shore 80A à 95A et 55D à 65D.

**11.** Dispositif (1) selon l'une des revendications précédentes, **caractérisé en ce que** les couches barrières (19) anti-odeurs présentent, en liaison avec les couches supports (24), des épaisseurs de paroi respectives comprises entre 5 et 20 $\mu$m.

**12.** Dispositif (1) selon l'une des revendications précédentes, **caractérisé en ce qu'**un ou plusieurs composants du dispositif de type cathéter (1) sont collés ensemble, par exemple en permettant à deux zones de surface des composants à assembler, zones de surface qui sont adjacentes lors de l'assemblage, d'être collées avec un solvant, en particulier avec un solvant tel que la cyclohexanone ou le tétrahydrofurane.

**13.** Dispositif (1) selon l'une des revendications précédentes, **caractérisé en ce qu'**au moins une couche barrière (19), en particulier une couche barrière (19) en EVOH, est liée à une couche (21) adjacente en PA, en particulier sans couche de liaison liante favorisant l'adhésion.

**14.** Dispositif (1) selon l'une des revendications précédentes, **caractérisé en ce qu'**au moins une couche support (24), en particulier une couche support (24) en PUR, est liée à une couche (21) adjacente en PA, en particulier sans couche de liaison liante favorisant l'adhésion.

**15.** Dispositif (1) selon l'une des revendications précédentes, **caractérisé en ce qu'**une couche (21) en PA est liée à une couche à base de PE (23) par l'intermédiaire d'un agent adhésif (22) en tant que couche intermédiaire.

**16.** Dispositif (1) selon l'une des revendications précédentes, **caractérisé en ce que** l'épaisseur (totale) $d_T$ de la couche support (24) ou de toutes les couches supports (24) est égale ou supérieure à dix fois l'épaisseur (totale) $d_B$ de la couche barrière (19) ou de toutes les couches barrières (19) :

$$d_T \geq 10 * d_B,$$

ou **en ce que** l'épaisseur (totale) $d_T$ de la couche support (24) ou de toutes les couches supports (24) est égale ou supérieure à vingt fois l'épaisseur (totale) $d_B$ de la couche barrière (19) en EVOH ou PVDC ou de toutes les couches barrières (19) en EVOH et/ou PVDC :

$$d_T \geq 20 \ {}^*d_B,$$

ou **en ce que** l'épaisseur (totale) $d_T$ de la couche support (24) ou de toutes les couches supports (24) est égale ou supérieure à quarante fois l'épaisseur (totale) $d_B$ de la couche barrière (19) en EVOH ou PVDC ou de toutes les couches barrières (19) en EVOH et/ou PVDC :

$$d_T \geq 40 \ {}^*d_B.$$

Fig.1

Fig.2

Fig.3

Fig.4a

Fig.4b

Fig.4c

Fig.4d

Fig.4e

14    17

17a 17b

Fig.4f

17a

Fig.5

18  19  20

Fig.6

21  19  20,18

Fig.7

18  19  18

Fig.8

19    23
      21
      21

Fig.9

18,20    19a

Fig.10a

Fig.10b

Fig.10c

R

25a$^{(3)}$

5$^{(3)}$

25b$^{(3)}$

25c$^{(3)}$

4$^{(3)}$

4a$^{(3)}$

3$^{(3)}$

26

Fig.10d

5$^{(4)}$

10$^{(4)}$

6$^{(4)}$

Fig.10e

5$^{(5)}$

26$^{(5)}$

6$^{(5)}$

Fig.10f

13a(6)

5(6)

15(6)

14(6)

13(6)

Fig.10g

5(7)

15(7)

13(7)  13a(7)

Fig.10h

5(8)

14(8)

13(8)

15(8)

13a(8)

Fig.10i

Fig.10j

Fig.10k

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014358126 A1
- US 6033390 A
- US 2013060212 A1
- US 2015282978 A1
- WO 2013056013 A1
- WO 2013026564 A
- WO 2009144028 A